# EUROPÄISCHE PATENTSCHRIFT

(11) **EP 1 813 597 B1**
(45) Veröffentlichungstag und Bekanntmachung des Hinweises auf die Patenterteilung: **20.04.2011**
(21) Anmeldenummer: 07000738.0
(22) Anmeldetag: 16.01.2007
(51) Int. Cl.: C07C 209/78, C07C 209/84, C07C 263/10

(54) **Verfahren zur Herstellung von Di- und Polyaminen der Diphenylmethanreihe**
Method for producing di- and polyamides in the diphenylmethane family
Procédé destiné à la fabrication de di- et polyamines de la série diphénylméthane

(30) Priorität: 28.01.2006 DE 102006004041
(43) Veröffentlichungstag der Anmeldung: 01.08.2007
(73) Patentinhaber: Bayer MaterialScience AG, 51368 Leverkusen (DE)
(72) Erfinder: Kegenhoff, Berthold, Dr., 47839 Krefeld (DE); Adamson, Richard, 42799 Leichlingen (DE); Uchdorf, Rudolf, 200001 Shangai (CN); Pohl, Fritz, Dr., 25541 Brunsbüttel (DE)

(56) Entgegenhaltungen:
- EP-A2- 1 344 766
- WO-A-99/40059
- US-A- 5 286 760

## Beschreibung

Die Erfindung betrifft ein Verfahren zur Herstellung von Gemischen von Di- und Polyaminen der Diphenylmethanreihe (MDA) enthaltend weniger als 1000 ppm an Wasser und weniger als 200 ppm an Anilin durch Umsetzung von Anilin und Formaldehyd in Gegenwart saurer Katalysatoren und anschließende Abtrennung des sauren Katalysators sowie anschließende destillative Abtrennung von Wasser und Anilin in einer wenigstens zweistufigen Destillation umfassend eine Entspannungsverdampfung und anschließende Kühlung.

Die Herstellung von Gemischen von Di- und Polyaminen der Diphenylmethanreihe (MDA) mit der Hauptkomponente Diaminodiphenylmethan (Diamine) durch Umsetzung von Anilin mit Formaldehyd in Gegenwart saurer Katalysatoren ist allgemein bekannt. Die Di- und Polyamingemische werden weit überwiegend zur Herstellung der entsprechenden Di- und Polyisocyanate (MDI) verwendet. Beispiele für kontinuierliche oder teilweise diskontinuierliche Verfahren sind in EP-A-1 344 766, US-A-5286760, EP-A-451442 und WO-A-99/ 40059 offenbart. Darin ist zwar erwähnt, dass nach Neutralisation der sauren Reaktionsmischung, Phasentrennung und ggf. Wäsche mit Wasser anhaftendes Wasser und überschüssiges Anilin destillativ, normalerweise im Vakuum entfernt wird. Sie geben aber keine Hinweise auf die Bedeutung der Gehalte an Wasser und Anilin für die weitere Verwendung der Di- und Polyamine zur Herstellung der entsprechenden Di- und Polyisocyanate. Auch sind in der Literatur keine Verfahren zum Erzielen dieser geringen Gehalte an Wasser und Anilin beschrieben. Auch gibt es keine Hinweise aus der Literatur, wie die Destillation der Di- und Polyamine unter Abtrennung von Anilin und Wasser energetisch optimiert werden kann. Ein weiteres Problemfeld ist die Rückführung des abgetrennten Anilins in die Reaktion, wobei gemäß EP-A-0283757 möglichst kein MDA in die Reaktion mit Formaldehyd rückgeführt werden soll.

Nun wurde gefunden, dass niedrige Gehalte an Wasser und Anilin in den Di- und Polyaminen von unter 1000 ppm Wasser und unter 200 ppm Anilin eine wichtige Voraussetzung für die Verarbeitung zu den entsprechenden Di- und Polyisocyanaten mit niedrigen Gehalten an Nebenprodukten ist. Denn höhere Gehalte an Wasser und Anilin in den Di- und Polyaminen führen im Prozess der Isocyanatherstellung zu Nebenreaktionen, z.B. zu erhöhten Gehalten an Eisen und leichtsiedenden Isocyanaten, die ihrerseits zur Bildung von chlorhaltigen Verunreinigungen, führen. Weiterhin wurde gefunden, dass diese sehr niedrigen Gehalte an Wasser und Anilin durch eine speziell abgestimmte Abfolge von Destillationsschritten und bevorzugt unter Nutzung der Wärme der erhaltenen Di- und Polyamine enthaltend weniger als 1000 ppm an Wasser und weniger als 200 ppm an Anilin erhalten werden können. Dabei enthält das als Destillat rückgewonnene Anilin, nach erfolgter Abtrennung von Wasser, bevorzugt weniger als 0,5 Gew. % an Di- und Polyaminen. Gegebenenfalls kann auch zusätzlich Fremdwärme, bevorzugt Dampf eines absoluten Drucks von 6 bar oder darunter eingesetzt werden, und so bei geringem Primärenergieeinsatz eine stabile Prozessführung erzielt werden. Ferner wurde gefunden, dass die Di- und Polyamine bei Lagerung bei hohen Temperaturen, besonders über 150°C, wieder Anilin abspalten, so dass eine Kühlung des Produktes nach der Destillation zum Erhalt der Qualität wesentlich ist.

Die Aufgabe der vorliegenden Erfindung bestand also darin, ein einfaches und mit niedrigem Energieverbrauch betreibbares Verfahren zur Herstellung von Di- und Polyaminen der Diphenylmethanreihe bereit zu stellen, das eine Herstellung der entsprechenden Di- und Polyisocyanate durch anschließende Phosgenierung unter reduzierter Nebenproduktbildung ermöglicht.

Die Erfindung betrifft ein Verfahren zur Herstellung von Di- und Polyaminen der Diphenylmethanreihe, bei dem
a) Anilin und Formaldehyd in Gegenwart eines sauren Katalysators zu einem Reaktionsgemisch enthaltend Di- und Polyamine umgesetzt wird, und
b) das Reaktionsgemisch enthaltend Di- und Polyamine neutralisiert wird, und
c) das neutralisierte Reaktionsgemisch enthaltend Di- und Polyamine in eine organische Phase enthaltend Di- und Polyamine und eine wässrige Phase aufgetrennt wird, und
d) aus der organischen Phase enthaltend Di- und Polyamine destillativ Wasser und Anilin abgetrennt werden,
   dadurch gekennzeichnet, dass
   d1) die Destillation in Schritt d) wenigstens eine Voreindampfstufe und wenigstens eine daran anschließende Destillationsstufe umfasst, und
   d2) in der Voreindampfstufe aus der organische Phase enthaltend Di- und Polyamine durch Entspannungsverdampfung Anilin und Wasser teilweise abgetrennt werden, und
   d3) in der anschließenden Destillationstufe noch enthaltenes Anilin und Wasser abgetrennt werden, wobei Di- und Polyamine enthaltend weniger als 1000 ppm an Wasser und weniger als 200 ppm an Anilin, bezogen auf das Gewicht der Di- und Polyamine, erhalten werden und
e) die Di- und Polyamine enthaltend weniger als 1000 ppm an Wasser und weniger als 200 ppm an Anilin anschließend abgekühlt werden.

In Schritt e) erfolgt die Abkühlung der Di- und Polyamine enthaltend weniger als 1000 ppm an Wasser und weniger als 200 ppm an Anilin bevorzugt durch Wärmeaustausch mit der in Schritt c) erhaltenen organischen Phase enthaltend Di- und Polyamine vor oder während deren Destillation in Schritt d), besonders bevorzugt vor oder während der Entspannungsverdampfung in der Voreindampfstufe (Schritt d2)) .

Bevorzugt enthält das in Schritt d3) als Destillat erhaltene Gemisch von Anilin und Wasser weniger als 0,5 Gew. % an Di- und Polyaminen, bezogen auf das Gewicht des Anilins bzw. des Anilinanteils im Gemisch. Bevorzugt wird aus diesem Gemisch von Anilin und Wasser dann Anilin abgetrennt und dabei dann ein Anilin enthaltend weniger als 0,5 Gew. % an Di- und Polyaminen erhalten.

Bevorzugt erfolgt die Kühlung der Di- und Polyamine enthaltend weniger als 1000 ppm an Wasser und weniger als 200 ppm an Anilin in Schritt e) im Wesentlichen unmittelbar nach deren Austritt aus der Destillation in Schritt d). Bevorzugt beträgt die Verweilzeit der Di- und Polyamine enthaltend weniger als 1000 ppm an Wasser und weniger als 200 ppm an Anilin nach deren Austritt aus der Destillationsstufe (Schritt d)) bei einer Temperatur von 180°C oder mehr maximal 30 min, besonders bevorzugt maximal 10 min.

Die Erfindung betrifft auch ein Verfahren zur Herstellung von Di- und Polyisocyanaten der Diphenylmethanreihe, bei dem Di- und Polyamine nach dem erfindungsgemäßen Verfahren hergestellt werden und anschließend mit Phosgen zu den entsprechenden Di- und Polyisocyanaten umgesetzt werden. Dabei kann die Phosgenierung nach einem der aus dem Stand der Technik bekannten Verfahren durchgeführt werden (z.B. DE-A-844 896 oder DE-A-198 17 691).

Die säurekatalysierte Kondensation von Anilin und Formaldehyd in Schritt a) kann nach einem Verfahren nach dem Stand der Technik durchgeführt werden. Dabei werden normalerweise Anilin und wässrige Formaldehydlösung bei Molverhältnissen im Bereich von 1,7 : 1 bis 20 : 1, bevorzugt 1,7: 1 bis 5 : 1 in Gegenwart eines sauren Katalysators, üblicherweise einer starken Mineralsäure wie Salzsäure, bei Einsatz von 0,001 bis 0,9, bevorzugt 0,08 bis 0,5 Mol Mineralsäure pro Mol Anilin kondensiert. Es können natürlich auch feste saure Katalysatoren wie in der Literatur beschrieben verwendet werden. Dabei kann das Formaldehyd in eine Mischung von Anilin und saurem Katalysator gegeben und die Reaktionslösung durch stufenweises Erhitzen ausreagiert werden. Alternativ kann auch Anilin und Formaldehyd zunächst vorreagieren und anschließend mit dem sauren Katalysator oder einem Gemisch von weiterem Anilin und saurem Katalysator versetzt werden, wonach die Reaktionslösung durch stufenweises Erhitzen ausreagiert wird. Diese Reaktion kann kontinuierlich oder diskontinuierlich nach einem der zahlreichen in der Literatur beschriebenen Verfahren ausgeführt werden.

In Schritt b) wird das Reaktionsgemisch enthaltend die Di- und Polyamine anschließend ggf. unter Zusatz von Wasser und / oder Anilin neutralisiert. Bevorzugt erfolgt die Neutralisation mit Natronlauge.

Anschließend wird in Schritt c) das neutralisierte Reaktionsgemisch enthaltend die Di- und Polyamine in eine organische Phase enthaltend Di- und Polyamine und eine wässrige Phase aufgetrennt. Dies kann durch die Zugabe von Anilin und / oder Wasser unterstützt werden. Wird die Phasentrennung durch Zugabe von Anilin und / oder Wasser unterstützt, so erfolgt deren Zugabe bevorzugt bereits unter intensivem Mischen in der Neutralisation. Dabei kann die Vermischung in Mischstrecken mit statischen Mischern, in Rührkesseln oder Rührkesselkaskaden oder aber in einer Kombination von Mischstrecken und Rührkessel erfolgen. Das neutralisierte und durch Zugabe von Anilin und / oder Wasser verdünnte Reaktionsgemisch wird anschließend bevorzugt einem Apparat zugeführt, der aufgrund seiner Konfiguration und / oder Einbauten besonders zur Auftrennung in eine organische Phase enthaltend MDA und eine wässrige Phase geeignet ist. Zum Einsatz kommen bevorzugt Scheideflaschen mit die Koalezenz der beiden Phasen unterstützenden Plattenpaketen als Einbauten.

Ggf. kann sich eine Wäsche der organischen Phase mit Wasser und neuerliche Abscheidung der Wasserphase zur Entfernung von Restgehalten an Salz (DE-A-2549890) anschließen.

In Schritt d) wird aus der in Schritt c) erhaltenen organischen Phase enthaltend Di- und Polyamine destillativ Wasser und Anilin abgetrennt. Die in Schritt c) erhaltene organische Phase hat bevorzugt eine Zusammensetzung, bezogen auf das Gewicht des Gemisches, von 5-15 Gew. % Wasser, und, je nach Einsatzverhältnissen von Anilin und Formaldehyd, 5-90 Gew. %, bevorzugt 5 - 40 Gew. % Anilin und 5-90 Gew.-%, bevorzugt 50 - 90 Gew. % Di- und Polyamine. Nach Austritt aus der Phasentrennung in Schritt c) hat die organische Phase enthaltend Di- und Polyamine üblicherweise eine Temperatur von 80 - 150°C.

Dabei ist es wesentlich für das erfindungsgemäße Verfahren, dass
d1) die Destillation in Schritt d) wenigstens eine Voreindampfstufe und wenigstens eine daran anschließende Destillationsstufe umfasst, und
d2) in der Voreindampfstufe aus der organische Phase enthaltend Di- und Polyamine durch Entspannungsverdampfung Anilin und Wasser teilweise abgetrennt werden, und
d3) in der anschließenden Destillationstufe noch enthaltenes Anilin und Wasser abgetrennt werden, wobei Di- und Polyamine enthaltend weniger als 1000 ppm an Wasser und weniger als 200 ppm an Anilin, bezogen auf das Gewicht der Di- und Polyamine, erhalten werden. Bevorzugt werden in der anschließenden Destillationsstufe in Schritt d3) sogar Di- und Polyamine mit Gehalten an Wasser von weniger als 500 ppm, besonders bevorzugt von weniger als 300 ppm, bezogen auf das Gewicht der Di- und Polyamine, erhalten. Weiterhin bevorzugt werden in der anschließenden Destillationsstufe in Schritt d3) sogar Di- und Polyamine mit Gehalten an Anilin von weniger als 50 ppm, besonders bevorzugt von weniger als 20 ppm, bezogen auf das Gewicht der Di- und Polyamine, erhalten. Dabei ist es vorteilhaft, die Gehalte an Wasser und Anilin in den Di- und Polyaminen so weit wie möglich abzusenken.

In Schritt e) werden die Di- und Polyamine enthaltend weniger als 1000 ppm an Wasser und weniger als 200 ppm an Anilin anschließend abgekühlt. Dies erfolgt bevorzugt durch Wärmeaustausch mit der organischen Phase enthaltend Di- und Polyamine vor oder während deren Destillation in Schritt d), bevorzugt vor oder während deren Entspannungsverdampfung vor oder während Schritt d2). Die Abkühlung kann jedoch auch durch ein Kühlmedium erfolgen.

Die Destillation in Schritt d) des erfindungsgemäßen Verfahrens wird nachstehend anhand der Figuren näher erläutert: Es zeigen
- Figur 1: ein Verfahrensfließbbild einer einstufigen Destillation, und
- Figur 2a: ein Verfahrensfließbbild einer einstufigen Destillation unter Zufuhr von Wasserdampf, und
- Figur 2b: ein Verfahrensfließbbild einer einstufigen Destillation unter Zufuhr von Wasserdampf, bei der zwischen dem Verdampfer und der Wasserdampfeinspeisung Trennstufen angeordnet sind, und
- Figur 3: ein Verfahrensfließbild einer zweistufigen Destillation umfassend eine Entspannungsverdampfung unter Zufuhr zusätzlicher Fremdenergie, und
- Figur 4: ein Verfahrensfließbild einer zweistufigen Destillation umfassend eine Entspannungsverdampfung unter Zufuhr von Abwärme aus der Destillation, und
- Figur 5: ein Verfahrensfließbild einer dreistufigen Destillation umfassend eine Entspannungsverdampfung unter Zufuhr zusätzlicher Fremdenergie und unter Zufuhr von Abwärme aus der Destillation
- Figur 6: ein Verfahrensfließbild einer dreistufigen Destillation umfassend eine Entspannungsverdampfung unter Zufuhr von Abwärme aus der Destillation und unter Zufuhr zusätzlicher Fremdenergie.

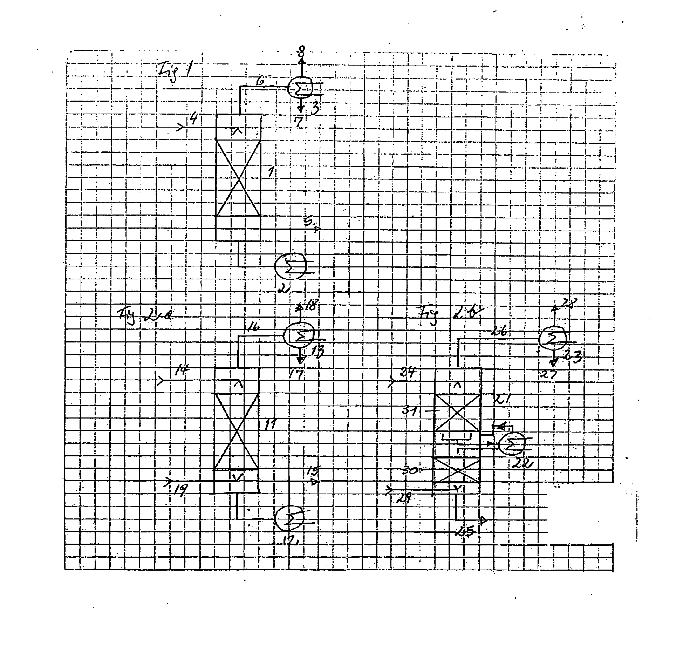

Figur 1 zeigt ein Verfahrensfließbbild einer einstufigen Destillation, in welcher Schritt d) des erfindungsgemäßen Verfahrens nicht durchgeführt werden kann. Darin wird der Strom 4 der in Schritt c) erhaltenen organischen Phase enthaltend Di- und Polyamine auf den Kopf einer Destillationskolonne 1 mit Sumpfverdampfer 2 und Kondensator 3 aufgegeben. Die Destillationskolonne 1 wird bei einem Kopfdruck von 2-100 mbar, bevorzugt 5-50 mbar und einer Sumpftemperatur von 200 - 300°C, bevorzugt 220 - 270°C und einer Brüdentemperatur von 20 - 200°C, bevorzugt 50 - 150°C betrieben. Die Kolonne hat vorteilhaft 2 bis 20, bevorzugt 3 bis 10 theoretische Trennstufen, die durch aus dem Stand der Technik bekannte Einbauten wie Trennböden oder Füllkörper, bevorzugt durch geordnete Packungen gebildet werden. Als Sumpfverdampfer 2 können beliebige Wärmeübertragungsapparate wie z. B. Umlaufverdampfer, Fallfilmverdampfer oder Einsteckbündel eingesetzt werden. Sie werden mit Heizdampf oder anderen Wärmeträgern, entsprechend der zu erreichenden Sumpftemperatur betrieben. Dem Sumpf oder dem Verdampfer 2 wird der Produktstrom 5 (Di- und Polyamine) entnommen und anschließend vorteilhaft auf eine Temperatur von 80 - 180°C, bevorzugt 90 - 150°C, besonders bevorzugt 100 - 120°C abgekühlt. Das abgetrennte Wasser-Anilin-Gemisch 6 wird im Kopfkondensator 3 kondensiert. Als Kopfkondensator können, je nach Kondensationstemperatur, bekannte Kondensatoren wie Luftkondensatoren, wassergekühlte oder solegekühlte Wärmetauscher, ggf. auch mehrere in Reihe verwendet werden. Die erforderliche Kondensationstemperatur ist dabei durch den gewählten Druck gegeben. Aus dem Kondensator 3 wird das Kondensat 7 in den Prozess zurückgeführt. Restgase 8 werden beispielsweise über ein Vakuumsystem als Abluft abgeführt.

Der Vorteil dieses Systems ist seine Einfachheit. Nachteilig ist jedoch die hohe Sumpftemperatur, welche die Zersetzung des Polyamins bewirken kann, und / oder der niedrige erforderliche Druck, der große Kolonnen und aufwendige Kondensationsmedien zur Folge hat. Nachteilig ist außerdem der hohe Verbrauch an Wärme auf hohem Temperaturniveau.

In einer alternativen Ausführungsform der einstufigen Destillation, in welcher Schritt d) des erfindungsgemäßen Verfahrens nicht durchgeführt werden kann, wird die Destillation unter Zufuhr von Wasserdampf betrieben. Diese Ausführungsform ist in den Figuren 2a und 2b gezeigt. In den Figuren 2a und 2b wird der Strom 14 bzw. 24 der in Schritt c) erhaltenen organischen Phase enthaltend Di- und Polyamine auf den Kopf einer Destillationskolonne 11, 21 aufgegeben, in die im Sumpf Wasserdampf eingeleitet wird (Wasserdampfdestillation). Die Destillationskolonne wird bei einem absoluten Druck von 20 - 1000 mbar, bevorzugt 50 - 200 mbar, einer Sumpftemperatur von 120 - 300°C, bevorzugt 180 - 260°C und einer Brüdentemperatur von 60 - 200°C, bevorzugt 80 - 150°C, betrieben. Die Kolonne 11, 21 hat bevorzugt 2 bis 20, bevorzugt 3 bis 10 theoretische Trennstufen, die durch aus dem Stand der Technik bekannte Einbauten wie Trennböden oder Füllkörper, bevorzugt durch geordnete Packungen gebildet werden. Im Sumpf der Kolonne 11, 21 wird Wasserdampf als Strom 19, 29 mit einer Temperatur von üblicherweise 80 - 350°C, bevorzugt 100 - 250°C, zugegeben. In einer bevorzugten Ausführung besitzt die Kolonne 11 einen zusätzlichen Sumpfverdampfer 12. In diesem Fall wird eine Menge von 10 - 200 kg Wasserdampf / t Di- und Polyamine, bevorzugt 20 - 100 kg/ t benötigt. Als Sumpfverdampfer 12 können beliebige Wärmeübertragungsapparate wie z. B. Umlaufverdampfer, Fallfilmverdampfer oder Einsteckbündel eingesetzt werden. Sie werden mit Heizdampf oder anderen Wärmeträgern, entsprechend der zu erreichenden Sumpftemperatur betrieben.

Die Kolonne kann auch ohne zusätzlichen Sumpfverdampfer 12 betrieben werden, allerdings ist dann eine Wasserdampfmenge von 1 - 10 t Wasserdampf / t Di- und Polyamine Produkt mit einer Temperatur von 280 - 350°C erforderlich.

Das Sumpfprodukt (Di- und Polyamine) wird im Sumpf der Kolonne als Strom 15, 25 entnommen und vorteilhaft auf eine Temperatur von 80 - 180°C, bevorzugt 90 - 150°C, besonders bevorzugt 100 - 120°C abgekühlt. Das abgetrennte Wasser-Anilin-Gemisch 16, 26 wird im Kopfkondensator 13, 23 kondensiert. Als Kopfkondensator können wiederum übliche Kondensatoren verwendet werden, die natürlich zusätzlich zu dem Wasser und Anilin aus der Rohlösung den zugegebenen Wasserdampf 19, 29 kondensieren müssen. Als Kopfkondensator können ebenfalls bekannte Kondensatoren wie Luftkondensatoren, wassergekühlte oder solegekühlte Wärmetauscher, ggf. auch mehrere in Reihe verwendet werden. Die erforderliche Kondensationstemperatur ist wiederum durch den gewählten Druck gegeben. Aus dem Kondensator 13, 23 wird das Kondensat 17, 27 in den Prozess zurückgeführt, Restgase 18, 28 werden bevorzugt über ein Vakuumsystem als Abluft abgeführt.

In einer bevorzugten Variante dieser zweiten Ausführungsform, die in Figur 2 b gezeigt ist, werden Dampfeinspeisung 29 und Verdampfer 22 an der Kolonne 21 so angeordnet, dass sich zwischen beiden ein Teil der Trennstufen 30 der Kolonne befinden. Die Flüssigkeit wird also auf den oberen Teil der Kolonneneinbauten 31 mit mindestens 1 - 5 theoretischen Trennstufen geführt, dann über den Verdampfer 22 geführt, wobei von dort Brüden wie Flüssigkeit wieder in die Kolonne 21 geleitet werden. Dieser zusätzliche Verdampfer kann z. B. als Umlaufverdampfer, Fallfilmverdampfer oder Einsteckbündel in die Kolonne eingebaut oder als außenliegender Verdampfer durch entsprechende Flüssigkeitsentnahmen aus der Kolonne und Rückführungsleitungen der Brüden und Flüssigkeit an die Kolonne angebunden sein.

Nach Austritt aus dem Verdampfer wird dann die Flüssigkeit auf den unteren Teil der Kolonneneinbauten 30 geleitet, unterhalb derer die Dampfeinspeisung 29 angebracht ist. Somit werden die Trennstufen 31 der Kolonne, die ebenfalls 2 bis 20, bevorzugt 3 bis,10 theoretischen Böden entsprechen und durch aus dem Stand der Technik bekannte Einbauten erzielt werden, in zwei Betten aufgeteilt, wobei das obere Bett bevorzugt 10 bis 70 % der gesamten Trennstufen, mindestens aber 1 Trennstufe besitzt.

Der Vorteil der anhand der Figuren 2a und 2b erläuterten Ausführungsformen gegenüber der in Figur 1 gezeigten Ausführungsform ist die niedrigere Sumpftemperatur bzw. der mögliche höhere Druck, der den Einsatz kleinerer Kolonnen ermöglicht und die Kondensation mit Luft oder Wasser ermöglicht. Nachteilig ist jedoch die große Menge an erzeugtem Brüdenkondensat und anfallendem Wasser, der hohe Verbrauch an Wärme auf hohem Temperaturniveau sowie ein hoher Wassergehalt des Sumpfproduktes.

Zur weiteren Erniedrigung des Wassergehaltes des Sumpfproduktes kann dieses darum zweckmäßigerweise anschließend durch Erhitzen und ggf. Strippen mit Inertgas nachbehandelt werden.

Das erfindungsgemäße Verfahren weist daher in Schritt d) einen wenigstens zweistufigen Destillationsschritt auf, der eine Voreindampfstufe mit Entspannungsverdampfung und eine nachfolgende Destillationsstufe aufweist, und bei dem der Energieinhalt der Ströme zur Beheizung bzw. Kühlung genutzt wird, was naturgemäß bei großen Anlagen und/ oder hohen Anilin- und Wassergehalten der Rohlösungen besonders vorteilhaft ist.

Dabei können ein, zwei oder mehrere Voreindampfschritte, ggf. auch als Stufendestillation mit Brüdennutzung, vorgesehen werden. Es hat sich als besonders vorteilhaft erwiesen, den Wärmeinhalt des aus der Destillationskolonne austretenden Sumpfproduktes (Di- und Polyamine enthaltend weniger als 1000 ppm an Wasser und weniger als 200 ppm an Anilin) als Energiequelle für eine solche Voreindampfstufe zu nutzen, wodurch gleichzeitig das Sumpfprodukt (Di- und Polyamine enthaltend weniger als 1000 ppm an Wasser und weniger als 200 ppm an Anilin) gekühlt wird. Durch die Abstimmung dieser Voreindampfschritte, vor allem bei Verwendung niederwertiger Energie (Abwärme auf niedrigem Temperaturniveau), kann der Einsatz von hochwertiger Energie (Wärme auf hohem Temperaturniveau) zur Erzielung des erfindungsgemäßen Produktes wesentlich verringert werden. Schließlich entlasten die in dem erfindungsgemäßen Verfahren enthaltenen Voreindampfstufen die Destillationskolonne erheblich, so dass nur noch wesentlich kleinere Kolonnen erforderlich sind bzw. vorhandene Kolonnen wesentlich höhere Kapazität bezogen auf die Di- und Polyamine erzielen können.

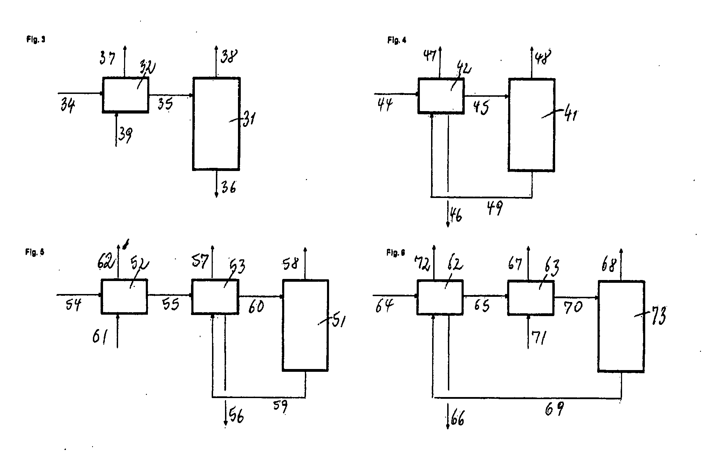

In Fig. 3 ist ein Verfahrensfließbild einer zweistufigen Destillation der in Schritt c) erhaltenen organischen Phase enthaltend Di- und Polyamine umfassend eine Voreindampfung (Entspannungsverdampfung) unter Zufuhr zusätzlicher Fremdenergie dargestellt, in welcher Schritt d) des erfindungsgemäßen Verfahrens durchgeführt werden kann. Dabei ist in Figur 3 eine Destillationskolonne 31 gezeigt, die bevorzugt wie eine der in den Figuren 1, 2a oder 2b dargestellten Destillationskolonnen enthaltend einen Verdampfer und einen Kondensator ausgeführt ist. Die Voreindampfung durch Entspannungsverdampfung wird in Apparat 32 durchgeführt. Die Voreindampfung durch Entspannungsverdampfung erfolgt dabei unter Nutzung des Wärmeinhalts der organischen Phase enthaltend Di- und Polyamine. Denn die Verdampfung des Brüdenstroms 37 erfolgt bei gleichzeitiger Abkühlung des Stroms 34 der organischen Phase enthaltend die Di- und Polyamine. Bevorzugt wird die Voreindampfung jedoch unter Zufuhr zusätzlicher Fremdenergie betrieben.

Die Voreindampfstufe besteht normalerweise aus Wärmetauscher und Dampf-Flüssigkeitsabscheider, wobei die Dampf-Flüssigkeitsabscheidung auch im Kopf der anschließenden Destillationskolonne erfolgen kann. In der Voreindampfstufe wird so bevorzugt 40 - 99,9 Gew.-%, bevorzugt 85 - 99 Gew.-% des Wasseranteils und 2-90 Gew.-%, bevorzugt 10-80 Gew.-% des Anilinanteils des Stroms 34 der organischen Phase enthaltend Di- und Polyamine verdampft. Sie kann unter Zufuhr von Abfallwärmen, wie z. B. heißem Wasser von z. B. 80 - 110°C oder, bevorzugt, mit Heizdampf von 1,1 bis 20, bevorzugt 1,2 bis 6 bar Druck und bei einer Temperatur von 40 bis 200°C, bevorzugt 80 bis 140°C sowie einem Druck von 20 - 2000 mbar, bevorzugt 50 bis 200 mbar betrieben werden. Der Zulaufstrom 34 (organischen Phase enthaltend Di- und Polyamine) wird dem Apparat 32 zur Entspannungsverdampfung zugeführt, die mit Wärmeträger (Heizmedium zur Zufuhr zusätzlicher Fremdenergie) 39 zusätzlich beheizt wird.

Der voreingedampfte Strom 35 der organische Phase enthaltend Di- und Polyamine mit einem verbleibenden Wasseranteil von 0,01 - 9 Gew. %, bevorzugt 0,1 - 2 Gew.% und Anilinanteil von 0,5 - 90 Gew. %, bevorzugt 1-35 Gew.%, jeweils bezogen auf das Gewicht der voreingedampften organischen Phase enthaltend Di- und Polyamine, wird anschließend der Destillationskolonne 31 zugeführt und dort in das Sumpfprodukt (Strom 36 der Di- und Polyamine enthaltend weniger als 1000 ppm an Wasser und weniger als 200 ppm an Anilin) und den Brüdenstrom 38 getrennt. Die Stufen 31 und 32 können bei unterschiedlichem oder gleichem Druck betrieben werden. Die Brüdenströme 37 und 38 können getrennt oder, vor allem bei gleichem Druck, auch gemeinsam, üblicherweise durch Kondensation, aufgearbeitet werden.

In Fig. 4 ist ein Verfahrensfließbild einer zweistufigen Destillation der in Schritt c) erhaltenen organischen Phase enthaltend Di- und Polyamine umfassend eine Voreindampfung (Entspannungsverdampfung) unter Zufuhr von Abwärme aus der Destillation dargestellt, in welcher Schritt d) des erfindungsgemäßen Verfahrens durchgeführt werden kann. Als Energiequelle zur Beheizung der Entspannungsverdampfung wird also der Wärmeinhalt des erzeugten Produktstroms (Strom der Di- und Polyamine enthaltend weniger als 1000 ppm an Wasser und weniger als 200 ppm an Anilin) verwendet und dieser dabei gleichzeitig abgekühlt. Dabei ist in Figur 4 eine Destillationskolonne 41 gezeigt, die bevorzugt wie eine der in den Figuren 1, 2a oder 2b dargestellten Destillationskolonnen enthaltend einen Verdampfer und einen Kondensator ausgeführt ist.

Die Voreindampfung durch Entspannungsverdampfung wird in Apparat 42 durchgeführt und enthält bevorzugt einen Wärmeaustauscher und einen Dampf-Flüssigkeitsabscheider. In der Voreindampfung wird bevorzugt 50 - 99,5 Gew.-%, besonders bevorzugt 70 - 98,5 Gew.-% des Wasseranteils und bevorzugt 2-90 Gew.-%, besonders bevorzugt 5-70 Gew.-% des Anilinanteils aus dem Strom 44 der in Schritt c) erhaltenen organischen Phase enthaltend Di- und Polyamine verdampft. Die Voreindampfung wird produktseitig bevorzugt bei einer Temperatur von 50 - 180°C, bevorzugt 60 - 120°C sowie einem absoluten Druck von 20 - 2000, bevorzugt 50 bis 200 mbar betrieben. Der voreingedampfte Strom 45 der organischen Phase enthaltend Di- und Polyamine mit einem verbleibenden Wasseranteil von bevorzugt 0,03 - 7 Gew. %, besonders bevorzugt 0,1 - 3 Gew. %, und Anilinanteil von bevorzugt 3-90 Gew. %, besonders bevorzugt 5-38 Gew. %, jeweils bezogen auf das Gewicht des Stroms 45, wird anschließend der Destillationskolonne 41 zugeführt und dort in das Sumpfprodukt (Strom 49 der Di- und Polyamine enthaltend weniger als 1000 ppm an Wasser und weniger als 200 ppm an Anilin) und den Brüdenstrom 48 getrennt.

Das heiße Sumpfprodukt 49 mit einer Produkttemperatur von bevorzugt 120 - 300°C, besonders bevorzugt 180 - 260°C wird nun als Heizmittel dem Wärmeaustauscher des Apparats 42 der Voreindampfstufe zugeführt, wodurch es um 20 - 200°C, bevorzugt 60 - 160°C abgekühlt wird und dann als gekühltes Sumpfprodukt 46 mit einer Temperatur von bevorzugt 80 - 180°C, besonders bevorzugt 90 - 150°C, ganz besonders bevorzugt 100 - 120°C abgeführt wird. Die Stufen 41 und 42 können bei unterschiedlichem oder gleichem Druck betrieben werden. Die Brüdenströme 47 und 48 können getrennt oder, vor allem bei gleichem Druck, auch gemeinsam, üblicherweise durch Kondensation, aufgearbeitet werden.

In Fig. 5 ist ein Verfahrensfließbild einer dreistufigen Destillation umfassend eine zweistufige Entspannungsverdampfung unter Zufuhr zusätzlicher Fremdenergie und unter Zufuhr von Abwärme aus der Destillation dargestellt, in welcher Schritt d) des erfindungsgemäßen Verfahrens durchgeführt werden kann.

Dabei kann die erste Voreindampfungsstufe 52 eine reine Entspannungsverdampfung unter ausschließlicher Nutzung des Wärmeinhalts des Stroms 54 der in Schritt c) erhaltenen organischen Phase enthaltend Di- und Polyamine sein, wird aber wie in Figur 5 gezeigt bevorzugt unter Zufuhr zusätzlicher Fremdenergie betrieben. Die Fremdenergie kann beispielsweise Dampf niedrigen Drucks oder andere Formen von bevorzugt niederwertiger Energie wie z. B. heißes Wasser sein. In der zweiten Voreindampfstufe 53 wird als Energiequelle für die weitere Entspannungsverdampfung des aus der ersten Voreindampfstufe 52 erhaltenen Stroms 55 der voreingedampften organischen Phase enthaltend Di- und Polyamine der Wärmeinhalt des aus der Destillationskolonne 51 erhaltenen Produktstroms (Strom 59 der Di- und Polyamine enthaltend weniger als 1000 ppm an Wasser und weniger als 200 ppm an Anilin) genutzt.

Die in Figur 5 dargestellte Destillationsstufe 51 ist dabei bevorzugt wie eine der in den Figuren 1, 2a oder 2b dargestellten Destillationskolonnen enthaltend einen Verdampfer und einen Kondensator ausgeführt. Die Voreindampfstufe 52 ist dagegen eine Voreindampfungsstufe, welche üblicherweise einen Wärmeaustauscher und einen Dampf-Flüssigkeitsabscheider umfasst. In der Voreindampfstufe 52 wird bevorzugt 40 - 99,9 Gew.-%, bevorzugt 85 - 99 Gew.-% des Wasseranteils und 2 - 90 Gew.-%, bevorzugt 10 - 80 Gew.-% des Anilinanteils, der im Strom 54 der in Schritt c) erhaltenen organischen Phase enthaltend Di- und Polyamine enthalten ist, verdampft. Der Zulauf 54 der organischen Phase enthaltend Di- und Polyamine wird der Voreindampfstufe 52 zugeführt, die mit einem Strom 61 eines Heizmediums (bevorzugt Abfallwärme wie z.B. heißes Wasser von z.B. 80 - 110°C oder Heizdampf von 1,1 bis 20, bevorzugt 1,2 bis 6 bar Druck) und produktseitig bei einer Temperatur von 40 bis 160°C, bevorzugt 80 bis 105°C sowie einem absoluten Druck von 20 - 2000 mbar, bevorzugt 50 bis 200 mbar betrieben werden kann. Die Abfuhr des abgekühlten Stroms des Heizmediums ist in Figur 5 nicht gezeigt.

Der voreingedampfte Strom 55 der organischen Phase enthaltend Di- und Polyamine mit einem verbleibenden Wasseranteil von bevorzugt 0,01 - 9 Gew. %, besonders bevorzugt 0,1 - 2 Gew.% und Anilinanteil von bevorzugt 0,5 - 90 Gew. %, besonders bevorzugt 5 - 35 Gew.%, jeweils bezogen auf das Gewicht des Stroms 55, wird der zweiten Voreindampfstufe 53 zugeführt, die üblicherweise ebenfalls einen Wärmeaustauscher und einen Dampf-Flüssigkeitsabscheider enthält. Sie wird produktseitig bei einer Temperatur von bevorzugt 100 - 200°C, besonders bevorzugt 130 - 180°C sowie einem absoluten Druck von bevorzugt 20 - 2000 mbar, besonders bevorzugt 50 bis 200 mbar betrieben. Der so erhaltene voreingedampfte Strom 60 der organischen Phase enthaltend Di- und Polyamine mit einem Wasseranteil von bevorzugt 50-500 ppm und einem Anilinanteil von bevorzugt 1 - 20 Gew. % , besonders bevorzugt von 3-12 Gew.%, jeweils bezogen auf das Gewicht des Stroms 60, wird nun der Destillationskolonne 51 zugeführt und dort in das Sumpfprodukt (Strom 59 der Di- und Polyamine enthaltend weniger als 1000 ppm an Wasser und weniger als 200 ppm an Anilin) und den Brüdenstrom 58 aufgetrennt. Der heiße Strom 59 des Sumpfprodukts mit einer produktseitigen Temperatur von bevorzugt 120 - 300°C, besonders bevorzugt 180 - 260°C wird nun als Heizmittel dem Wärmetauscher der zweiten Voreindampfstufe 53 zugeführt, wodurch es um 20 - 180°C abgekühlt wird und dann als gekühltes Sumpfprodukt 56 (Di- und Polyamine) mit einer Temperatur von bevorzugt 80 - 180°C, besonders bevorzugt 90 - 150°C, ganz besonders bevorzugt 100 - 120°C abgeführt wird. Die Stufen 51, 52 und 53 können bei unterschiedlichem oder gleichem Druck betrieben werden. Die Brüdenströme 82, 57 und 58 können getrennt oder, vor allem bei gleichem Druck, auch gemeinsam, üblicherweise durch Kondensation, aufgearbeitet werden.

In Fig. 6 ist ein Verfahrensfließbild einer dreistufigen Destillation umfassend eine Entspannungsverdampfung unter Zufuhr von Abwärme aus der Destillation und unter Zufuhr zusätzlicher Fremdenergie dargestellt, in welcher Schritt d) des erfindungsgemäßen Verfahrens durchgeführt werden kann.

Dabei ist die erste Voreindampfungsstufe 62 eine reine Entspannungsverdampfung, die unter Nutzung des Wärmeinhalts des in der Destillation aus der Destillationskolonne 73 erhaltenen Sumpfprodukts (Strom 69 der Di- und Polyamine enthaltend weniger als 1000 ppm an Wasser und weniger als 200 ppm an Anilin) durch Wärmeaustausch betrieben wird. In der zweiten Voreindampfstufe 63 wird als Energiequelle für die weitere Entspannungsverdampfung Fremdenergie genutzt. Die Fremdenergie kann beispielsweise Dampf niedrigen Drucks oder andere Formen von bevorzugt niederwertiger Energie wie z. B. heißes Wasser sein.

Die in Figur 6 dargestellte Destillationsstufe 73 ist dabei bevorzugt wie eine der in den Figuren 1, 2a oder 2b dargestellten Destillationskolonnen enthaltend einen Verdampfer und einen Kondensator ausgeführt. Die Voreindampfstufe 62 ist dagegen eine Voreindampfungsstufe, welche üblicherweise einen Wärmeaustauscher und einen Dampf-Flüssigkeitsabscheider umfasst. In der Voreindampfstufe 62 wird bevorzugt 60 - 99 Gew.-% des Wasseranteils und bis zu 80 Gew.-% des Anilinanteils, der im Strom 64 der in Schritt c) erhaltenen organischen Phase enthaltend Di- und Polyamine enthalten ist, verdampft.

Sie wird produktseitig bei einer Temperatur von bevorzugt 40 bis 180°C, besonders bevorzugt 50 bis 110°C sowie einem absoluten Druck von bevorzugt 20 - 2000 mbar, besonders bevorzugt 50 bis 200 mbar betrieben. Der Zulauf 64 der organischen Phase enthaltend Di- und Polyamine wird der Voreindampfstufe 62 zugeführt, darin voreingedampft, und der erhaltene Strom 65 der voreingedampften organischen Phase enthaltend Di- und Polyamine mit einem Wasseranteil von bevorzugt 0,5 - 9 Gew.%, besonders bevorzugt 0,1 - 2 Gew.% und einem Anilinanteil von bevorzugt 2 - 80 %, besonders bevorzugt 10 - 35 Gew.% jeweils bezogen auf das Gewicht des Stroms 65, der zweiten Voreindampfstufe 63 zugeführt. Die zweite Voreindampfstufe 63 umfasst üblicherweise ebenfalls einen Wärmeaustauscher und einen Dampf-Flüssigkeitsabscheider. Sie wird bevorzugt mit einem Strom 71 eines Heizmediums (bevorzugt Abfallwärme wie z. B. heißes Wasser von z. B. 80 - 110°C oder Heizdampf von 1,1 bis 20, bevorzugt 1,2 bis 6 bar Druck) und produktseitig bei einer Temperatur von bevorzugt 100 bis 200°C, besonders bevorzugt 130 bis 180°C, sowie einem absoluten Druck von bevorzugt 20 bis 2000 mbar, besonders bevorzugt 50 bis 200 mbar betrieben. Die Abfuhr des abgekühlten Stroms des Heizmediums ist in Figur 6 nicht gezeigt.

Der voreingedampfte Strom 70 der organischen Phase enthaltend Di- und Polyamine aus der zweiten Voreindampfstufe 63 mit einem Wasseranteil von bevorzugt unter 1 Gew. %, besonders bevorzugt 100 bis 800 ppm und einem Anilinanteil von bevorzugt 1 - 50 Gew. %, besonders bevorzugt 4 bis 20 Gew. %, jeweils bezogen auf das Gewicht des Stroms 70, wird nun der Destillationskolonne 73 zugeführt und dort in das Sumpfprodukt (Strom 69 der Di- und Polyamine enthaltend weniger als 1000 ppm an Wasser und weniger als 200 ppm an Anilin) und den Brüdenstrom 68 getrennt. Der heiße Sumpfproduktstrom 69 mit einer Temperatur von bevorzugt 120 - 300°C, besonders bevorzugt 180 - 260°C wird nun als Heizmittel dem Wärmeaustauscher der ersten Voreindampfstufe 62 zugeführt, wodurch es um bevorzugt 20 - 180°C abgekühlt wird und dann als gekühltes Sumpfprodukt 66 mit einer Temperatur von bevorzugt 80 - 180°C, besonders bevorzugt 90 - 150°C, ganz besonders bevorzugt 100 - 120°C abgeführt wird. Die Stufen 62, 63 und 73 können bei unterschiedlichem oder gleichem Druck betrieben werden. Die Brüdenströme 72, 67 und 68 können getrennt oder, vor allem bei gleichem Druck, auch gemeinsam, üblicherweise durch Kondensation, aufgearbeitet werden.

In den beschriebenen Ausführungsformen, in welcher Schritt d) des erfindungsgemäßen Verfahrens durchgeführt werden kann, wird mit unterschiedlich hohem Energieeinsatz als Sumpfprodukt das Gemisch von Di- und Polyaminen der Diphenylmethanreihe mit Gehalten an Wasser von unter 1000, bevorzugt unter 500 und besonders bevorzugt unter 300 ppm sowie Gehalten an Anilin von unter 200, bevorzugt unter 50, besonders bevorzugt unter 20 ppm, bezogen auf das Gewicht des Gemisches von Di- und Polyaminen, erhalten.

In den nachfolgenden Beispielen wird die Erfindung weiter erläutert:

### Beispiele:

### Beispiel 1

### Herstellung der organischen Phase enthaltend Di- und Polyamine (Schritte a) bis c))

In einem Rührgefäß werden 2600 g Anilin bei 25°C mit 1000 g Formalin (30 gew. %ige wässrige Lösung von Formaldehyd) unter Rühren intensiv vermischt, wobei sich die Mischung auf 60°C erwärmt. Man stellt den Rührer ab und trennt die sich oben abscheidende Wasserphase ab. Anschließend mischt man unter erneutem Rühren und Kühlen 680 g 30 gew. %ige wässrige Salzsäure zu, wobei eine Temperatur von 45°C gehalten wird. Nach 15 min weiterem Rühren bei dieser Temperatur wird die Kühlung durch Heizung ersetzt und die Mischung im Lauf von 120 min unter 5 bar Druck gleichmäßig auf 140°C aufgeheizt und danach 15 min bei dieser Temperatur gehalten.

Anschließend wird die Mischung auf 100°C abgekühlt, auf Normaldruck entspannt und durch Zugabe von 540 g 50 gew. %ige wässrige Natronlauge unter Rühren neutralisiert. Nach Abstellen des Rührers lässt man die Phasen absitzen und saugt die untere Salzwasserphase ab. Danach vermischt man die organische Phase bei 100°C mit 300 g Wasser, lässt erneut die Phasen sich trennen und trennt die oben aufschwimmende Wasserphase ab. Man erhält 3005 g der organischen Phase enthaltend Di- und Polyamine von 100°C der Zusammensetzung:
1160 g = 39 Gew. % Diaminodiphenylmethan (Zweikern-MDA, Diamin-Isomerengemisch)
600 g = 20 Gew. % Polyamine (drei- und höherkemiges MDA)
935 g = 31 Gew. % Anilin
310 g = 10 Gew. % Wasser

### Beispiel 2 (nicht erfindungsgemäß)

### Aufarbeitung der organischen Phase enthaltend Di- und Polyamine (ohne Voreindampfung)

Es wird eine Apparatur nach Fig. 2b bestehend aus einer Kolonne 21 mit Durchmesser 80 mm, einem oberen Bett mit einer Strukturpackung Mellapak 250X von 1659 mm Höhe (4 theoretische Trennstufen), einem darunter befindlichen, mit 30 bar Dampf beheizten Fallfilmverdampfer und einem unteren Bett mit einer Strukturpackung Mellapak 250X von 1659 mm Höhe (4 theoretische Trennstufen) eingesetzt. Die Kolonne wird bei 60 mbar betrieben. Die in Beispiel 1 erhaltene organische Phase enthaltend Di- und Polyamine wird mit 400 g/ min auf die Kolonne aufgegeben und im Verdampfer auf 220°C aufgeheizt, wobei Wasser und Anilin abdestillieren. Unter dem unteren Bett werden 12 g/min Wasserdampf mit einer Temperatur von 230°C eingespeist. Die Brüden werden an einem mit Wasser gekühlten Kondensator kondensiert. Das die Kolonne mit 205°C verlassende Sumpfprodukt wird durch Wasserkühlung auf 100°C abgekühlt und hat einen Wassergehalt von 285 ppm sowie einen Anilingehalt von 12 ppm. Der Verbrauch an Heizdampf mit 30 bar Druck beträgt 490 g/ kg Produkt (Di- und Polyamine).

### Beispiel 3 (erfindungsgemäß)

### Aufarbeitung der organischen Phase enthaltend Di- und Polyamine (mit Voreindampfung)

Es wird eine Apparatur nach Fig. 5 eingesetzt, welche eine erste Voreindampfstufe 52, enthaltend einen Wärmetauscher, der mit 1,5 bar Dampf (Abwärme z. B. aus der Entspannung von 30 bar Dampfkondensat) beheizt wird, und einen Gas-Flüssig-Abscheider enthält, ferner eine zweite Voreindampfstufe 53, enthaltend einen Wärmetauscher, der mit dem heißen Produktablauf der Destillationskolonne 51 beheizt wird, und einen Gas-Flüssig-Abscheider enthält, und schließlich die Destillationskolonne 51 mit Durchmesser 80 mm, einem oberen Bett mit einer Strukturpackung Mellapak 250X von 1659 mm Höhe (4 theoretische Trennstufen), einem darunter befindlichen, mit 30 bar Dampf beheizten Fallfilmverdampfer und einem unteren Bett mit einer Strukturpackung Mellapak 250X von 1659 mm Höhe (4 theoretische Trennstufen) enthält. Die erste Voreindampf stufe 52 wird bei 80 mbar absolutem Druck, die zweite Voreindampfstufe 53 bei 70 mbar absolutem Druck und die Destillationskolonne 51 bei 60 mbar absolutem Druck betrieben. Die in Beispiel 1 erhaltene organische Phase enthaltend Di- und Polyamine wird mit 1600 g/ min in die erste Voreindampfstufe 52 gegeben, worin sich eine Temperatur von 97°C einstellt. Der Sumpf aus dem Abscheider dieser Stufe fließt dann in die zweite Voreindampfungsstufe 53, worin sich eine Temperatur von 141°C einstellt. Der Sumpf aus dem Abscheider dieser Stufe wird nun auf die Destillationskolonne 51 aufgegeben und im Verdampfer auf 220°C aufgeheizt, wobei verbleibendes Wasser und Anilin abdestillieren. Unter dem unteren Bett werden 47 g/min Wasserdampf mit einer Temperatur von 120°C eingespeist. Die Brüden aller drei Stufen werden getrennt an mit Wasser gekühlten Kondensatoren kondensiert. Dabei enthält das Kondensat, bezogen auf den Anilinanteil, 0,13 Gew. % MDA. Das die Kolonne mit 210°C verlassende Sumpfprodukt wird durch den Wärmetauscher der zweiten Voreindampfungsstufe 53 auf 118°C abgekühlt und hat einen Wassergehalt von 285 ppm sowie einen Anilingehalt von 12 ppm. Der Verbrauch an Heizdampf mit 30 bar Druck beträgt 120g/ kg Produkt (Di- und Polyamine), der Verbrauch an Heizdampf mit 1,5 bar Druck (Abwärme) 230g/ kg Produkt (Di- und Polyamine).

## Patentansprüche

1. Verfahren zur Herstellung von Di- und Polyaminen der Diphenylmethanreihe, bei dem
a) Anilin und Formaldehyd in Gegenwart eines sauren Katalysators zu einem Reaktionsgemisch enthaltend Di- und Polyamine umgesetzt wird, und
b) das Reaktionsgemisch enthaltend Di- und Polyamine neutralisiert wird, und
c) das neutralisierte Reaktionsgemisch enthaltend Di- und Polyamine in eine organische Phase enthaltend Di- und Polyamine und eine wässrige Phase aufgetrennt wird, und
d) aus der organischen Phase enthaltend Di- und Polyamine destillativ Wasser und Anilin abgetrennt werden,
**dadurch gekennzeichnet, dass**
d1) die Destillation in Schritt d) wenigstens eine Voreindampfstufe und wenigstens eine daran anschließende Destillationsstufe umfasst, und
d2) in der Voreindampfstufe aus der organische Phase enthaltend Di- und Polyamine durch Entspannungsverdampfung Anilin und Wasser teilweise abgetrennt werden, und
d3) in der anschließenden Destillationstufe noch enthaltenes Anilin und Wasser abgetrennt werden, wobei Di- und Polyamine enthaltend weniger als 1000 ppm an Wasser und weniger als 200 ppm an Anilin, bezogen auf das Gewicht der Di- und Polyamine, erhalten werden und
e) die Di- und Polyamine enthaltend weniger als 1000 ppm an Wasser und weniger als 200 ppm an Anilin anschließend abgekühlt werden.

2. Verfahren nach Anspruch 1, bei dem die Abkühlung der Di- und Polyamine enthaltend weniger als 1000 ppm an Wasser und weniger als 200 ppm an Anilin in Schritt e) durch Wärmeaustausch mit der organischen Phase enthaltend Di- und Polyamine vor oder während deren Destillation in Schritt d) erfolgt.

3. Verfahren nach Anspruch 1 oder 2, bei dem die Voreindampfung der organische Phase enthaltend Di- und Polyamine in Schritt d2) durch mindestens zweistufige Entspannungsverdampfung und unter Zufuhr von Wärme erfolgt, die durch Wärmeaustausch der organischen Phase enthaltend Di- und Polyamine mit dem Strom der Di- und Polyamine enthaltend weniger als 1000 ppm an Wasser und weniger als 200 ppm an Anilin ausgetauscht wird.

4. Verfahren nach einem der Ansprüche 1 bis 3, bei dem das in Schritt d3) als Destillat erhaltene Gemisch von Anilin und Wasser weniger als 0,5 Gew. % an Di- und Polyaminen, bezogen auf das Gewicht des Anilins, enthält.

5. Verfahren zur Herstellung von Di- und Polyisocyanaten der Diphenylmethanreihe, bei dem Di- und Polyamine nach dem Verfahren nach Anspruch 1 hergestellt werden und anschließend mit Phosgen zu den entsprechenden Di- und Polyisocyanaten umgesetzt werden.

## Claims

1. Process for preparing di- and polyamines of the diphenylmethane series, in which
a) aniline and formaldehyde are reacted in the presence of an acidic catalyst to give a reaction mixture comprising di- and polyamines, and
b) the reaction mixture comprising di- and polyamines is neutralized, and
c) the neutralized reaction mixture comprising di- and polyamines is separated into an organic phase comprising di- and polyamines and an aqueous phase, and
d) water and aniline are removed by distillation from the organic phase comprising di- and polyamines,
**characterized in that**
d1) the distillation in step d) comprises at least one preliminary concentration stage and at least one subsequent distillation stage, and
d2) in the preliminary concentration stage, aniline and water are partly removed by flash evaporation from the organic phase comprising di- and polyamines, and
d3) aniline and water still present in the subsequent distillation stage are removed to obtain di- and polyamines containing less than 1000 ppm of water and less than 200 ppm of aniline, based on the weight of the di- and polyamines, and
e) the di- and polyamines containing less than 1000 ppm of water and less than 200 ppm of aniline are subsequently cooled.

2. Process according to Claim 1, in which the di- and polyamines containing less than 1000 ppm of water and less than 200 ppm of aniline are cooled in step e) by heat exchange with the organic phase comprising di- and polyamines before or during the distillation thereof in step d).

3. Process according to Claim 1 or 2, in which the preliminary concentration of the organic phase comprising di- and polyamines in step d2) is effected by at least two-stage flash evaporation and with supply of heat, which is exchanged by heat exchange of the organic phase comprising di- and polyamines with the stream of the di- and polyamines containing less than 1000 ppm of water and less than 200 ppm of aniline.

4. Process according to any of Claims 1 to 3, in which the mixture of aniline and water obtained as the distillate in step d3) contains less than 0.5% by weight of di- and polyamines based on the weight of the aniline.

5. Process for preparing di- and polyisocyanates of the diphenylmethane series, in which di- and polyamines are prepared by the process according to Claim 1 and then reacted with phosgene to give the corresponding di- and polyisocyanates

## Revendications

1. Procédé pour la préparation de di- et polyamines de la série diphénylméthane, dans lequel
a) on fait réagir de l'aniline et du formaldéhyde en présence d'un catalyseur acide, pour obtenir un mélange réactionnel contenant des di- et polyamines, et
b) on neutralise le mélange réactionnel contenant des di- et polyamines, et
c) on divise le mélange réactionnel neutralisé, contenant des di- et polyamines, en une phase organique contenant des di- et polyamines et une phase aqueuse, et
d) on sépare par distillation l'aniline et l'eau de la phase organique contenant des di- et polyamines,
**caractérisé en ce que**
d1) la distillation dans l'étape d) comprend au moins une étape de pré-évaporation et au moins une étape de distillation y faisant suite, et
d2) dans l'étape de pré-évaporation l'aniline et l'eau sont partiellement séparées de la phase organique contenant des di- et polyamines par évaporation par détente, et
d3) dans l'étape de distillation subséquente on sépare l'aniline et l'eau encore contenues, pour obtenir des di- et polyamines contenant moins de 1 000 ppm d'eau et moins de 200 ppm d'aniline, par apport au poids des di- et polyamines et
e) ensuite on refroidit les di- et polyamines contenant moins de 1 000 ppm d'eau et moins de 200 ppm d'aniline.

2. Procédé selon la revendication 1, dans lequel on effectue dans l'étape e) le refroidissement des di- et polyamines contenant moins de 1 000 ppm d'eau et moins de 200 ppm d'aniline par échange thermique avec la phase organique contenant des di- et polyamines, avant ou pendant leur distillation dans l'étape d).

3. Procédé selon la revendication 1 ou 2, dans lequel la pré-évaporation de la phase organique contenant des di- et polyamines s'effectue dans l'étape d2) par évaporation par détente en au moins deux stades et avec apport de chaleur, qui est échangée par échange thermique de la phase organique contenant des di- et polyamines avec le courant des di- et polyamines contenant moins de 1 000 ppm d'eau et moins de 200 ppm d'aniline.

4. Procédé selon l'une quelconque des revendications 1 à 3, dans lequel le mélange d'aniline et d'eau, obtenu en tant que distillat dans l'étape d3), contient moins de 0,5 % en poids de di- et polyamines, par rapport au poids de l'aniline.

5. Procédé pour la production de di- et polyisocyanates de la série diphénylméthane, dans lequel on prépare des di- et polyamines conformément au procédé selon la revendication 1 et ensuite on les fait réagir avec du phosgène pour obtenir les di- et polyisocyanates correspondants.
